# FASCICULE DE BREVET EUROPEEN

(11) **EP 0 935 979 B1**
(45) Date de publication et mention de la délivrance du brevet: **11.04.2007**
(21) Numéro de dépôt: 99400334.1
(22) Date de dépôt: 11.02.1999
(51) Int. Cl.: A61N 1/37

(54) **Dispositif médical implantable actif, notamment stimulateur cardiaque, défibrillateur et/ou cardioverteur, de type multisite**
Aktive implantierbare medizinische Vorrichtung, insbesondere mehrstelliger Herzstimulator, Defibrillator und/oder Kardiovertierer
Active implantable medical device, in particular multisite pacemaker, defibrillator and/or cardioverter

(30) Priorité: 13.02.1998 FR 9801788
(43) Date de publication de la demande: 18.08.1999
(73) Titulaire: ELA MEDICAL (Société anonyme), 92541 Montrouge (FR)
(72) Inventeur: Limousin, Marcel, 75014 Paris (FR)
(74) Mandataire: Dupuis-Latour, Dominique

(56) Documents cités:
- EP-A- 0 479 215
- EP-A- 0 653 225
- WO-A-86/05698
- WO-A-96/04956

## Description

L'invention concerne les "dispositifs médicaux implantables actifs" tels que définis par la directive 90/385/CEE du 20 juin 1990 du Conseil des communautés européennes, plus précisément les dispositifs stimulateurs cardiaques, défibrillateurs et/ou cardioverteurs permettant de délivrer au coeur des impulsions électriques de faible énergie pour le traitement des troubles du rythme cardiaque.

Elle concerne plus particulièrement les prothèses dites "multisite", c'est-à-dire les prothèses dans lesquelles des électrodes sont placées en une pluralité de sites respectifs distincts, avec au moins deux sites ventriculaires. Il peut s'agir d'une prothèse de type biventriculaire (double stimulation ventriculaire), triple chambre (stimulation atriale droi- ! te et double stimulation ventriculaire) ou même quadruple chambre (double stimulation atriale et double stimulation ventriculaire).

En effet, outre le traitement des troubles du rythme cardiaque, il a été proposé de traiter par stimulation les troubles de contraction myocardique observés chez des patients en insuffisance cardiaque, ces troubles pouvant être spontanés ou induits par une stimulation traditionnelle. On pourra notamment se référer à l'étude de J. C. Daubert et coll., *Stimucoeur,* Tome 25, n°3, 1997, pp. 170-176 qui fait le point des travaux sur ce sujet. Il a été notamment proposé de stimuler simultanément les ventricules gauche et droit, mais cette technique peut aussi bien s'appliquer à la double stimulation des oreillettes.

La vérification de la capture est une procédure en elle-même connue, par exemple d'après les WO-A-86 05698 et EP-A-0 479 215, mais dans le contexte d'un stimulateur "double chambre" classique, comprenant une électrode ventriculaire droite et une électrode auriculaire droite, c'est-à-dire où les deux électrodes sont placées dans deux cavités situées d'un même côté du coeur.

L'une des difficultés de la stimulation multisite est de garantir l'efficacité de la stimulation des différents sites. En particulier, on constate généralement entre le site droit et le site gauche dès seuils différentes, ce qui peut conduire à une stimulation défectueuse ou une détection erronée des ondes de dépolarisation (confusion entre une dépolarisation électrostimulée à l'endroit du site et une dépolarisation captée indirectement en provenance du site voisin).

L'un des buts de l'invention est de remédier à cette difficulté, grâce à un ajustement automatique de l'amplitude de stimulation des deux sites droit et gauche par rapport au "seuil d'entraînement", c'est-à-dire le niveau minimal permettant la capture, sur le site considéré, d'une dépolarisation consécutive à une impulsion de stimulation antérieure.

À cet effet, l'invention propose un dispositif du type multisite précité, c'est-à-dire dans lequel des électrodes sont placées en un site ventriculaire droit et en un site ventriculaire gauche (ou en un site auriculaire droit et en un site auriculaire gauche) , ces électrodes étant reliées à un circuit de recueil de signaux cardiaques pour détecter un potentiel de dépolarisation sur le site correspondant ainsi qu'à un circuit de stimulation pour appliquer si nécessaire une impulsion de stimulation sur ce même site. Le dispositif comporte des moyens pour ajuster l'amplitude de stimulation par rapport au seuil d'entraînement et des moyens pour fixer une période réfractaire totale comportant une période réfractaire absolue, pendant laquelle est inhibée toute détection, suivie d'une période réfractaire relative.

Le FR-A-2 680 093 (ELA Médical), auquel on pourra se référer pour de plus amples détails, décrit un dispositif pourvu d'un tel ajustement automatique de l'amplitude de stimulation par rapport au seuil d'entraînement. Cet ajustement est obtenu par réduction progressive du niveau de l'amplitude sur plusieurs cycles successifs, détection de la disparition de la capture, puis rétablissement de l'amplitude à un niveau légèrement supérieur au seuil correspondant à cette disparition.

Selon l'invention, la détection d'une dépolarisation pendant la période réfractaire relative marque l'absence d'une capture.

Très avantageusement, il est prévu des moyens pour ajuster l'instant de début de la période réfractaire relative, notamment par accroissement progressif de la durée de cette période réfractaire relative sur plusieurs cycles successifs jusqu'à détection d'une dépolarisation dans cette période réfractaire relative, sans modification de la durée de la période réfractaire totale.

De préférence, la durée de de la période réfractaire totale est constante et la durée initiale de la période réfractaire relative est nulle.

Il peut être prévu des moyens de détection préalable d'absence d'activité extrasystolique, conditionnant la mise en oeuvre des moyens d'ajustement de l'amplitude de stimulation, comportant notamment des moyens pour évaluer le temps maximal de dépolarisation synchrone des deux sites ventriculaires et/ou des deux sites atriaux.

Enfin, pour éviter des fausses détections, le dispositif comprend avantageusement des moyens de détection de bigéminisme, propres à inhiber l'ajustement de l'amplitude de stimulation même en cas de détection d'une dépolarisation pendant la période réfractaire relative.

D'autres caractéristiques et avantages de l'invention apparaîtront à la lecture de la description détaillée ci-dessous d'un exemple de mise en oeuvre de l'invention, en référence aux dessins annexés.
La figure 1 montre schématiquement la division de la période réfractaire en une partie absolue et une partie relative, avec une frontière variable.
Les figures 2 et 3 illustrent ensemble un organigramme de mise en oeuvre d'un algorithme d'ajustement automatique de l'amplitude de stimulation selon l'invention.

Le but de l'invention, comme on l'a indiqué plus haut, est de garantir l'efficacité de la stimulation des différents sites dans un stimulateur multisite, en tenant compte du fait que l'on constate en général des seuils différents entre le site droit et le site gauche.

Dans la description ci-dessous, on se référera à des sites ventriculaires (ondes R) droit et gauche, mais l'invention peut être parfaitement transposée au cas de deux sites atriaux voisins, notamment dans le cas des modèles dits "quadruple chambre" incluant une double stimulation atriale.

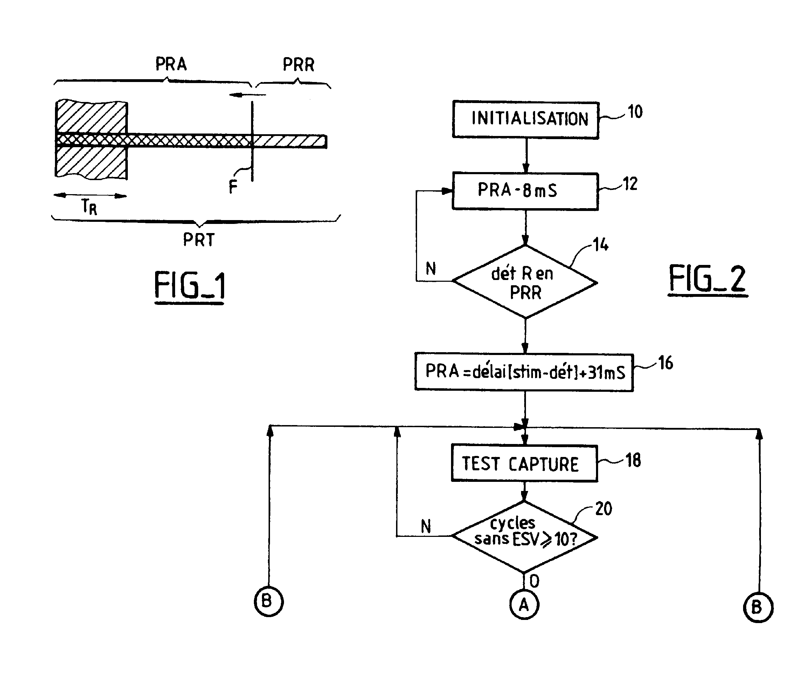

Pour ce faire, selon l'invention, on divise la période réfractaire totale PRT (période réfractaire post-ventriculaire - comme dans l'exemple ci-dessous - ou période réfractaire post-atriale), qui est un paramètre connu du stimulateur, généralement fixé à une valeur programmée, en deux sous-périodes, à savoir une période réfractaire absolue PRA, suivie d'une période réfractaire relative PRR (figure 1).

Pendant la période réfractaire absolue PRA, aucun signal ne sera détecté, quelle qu'en soit la nature et l'origine. Cette période réfractaire absolue doit être au moins égale au temps de récupération T_{R} des amplificateurs, qui est de l'ordre de 50 ms ; elle ne doit par contre pas être trop longue, au risque de ne pas détecter d'éventuelles extrasystoles ventriculaires, qui nécessitent la mise en oeuvre d'un algorithme d'analyse particulier.

En revanche, pendant la période réfractaire relative PRR, on autorisera une analyse de la dépolarisation ventriculaire dans le but particulier de l'ajustement des amplitudes de stimulation selon l'invention ; mais les dépolarisations détectées pendant cette période réfractaire relative ne serviront pas à la commande de la stimulation (car cette période reste une période réfractaire).

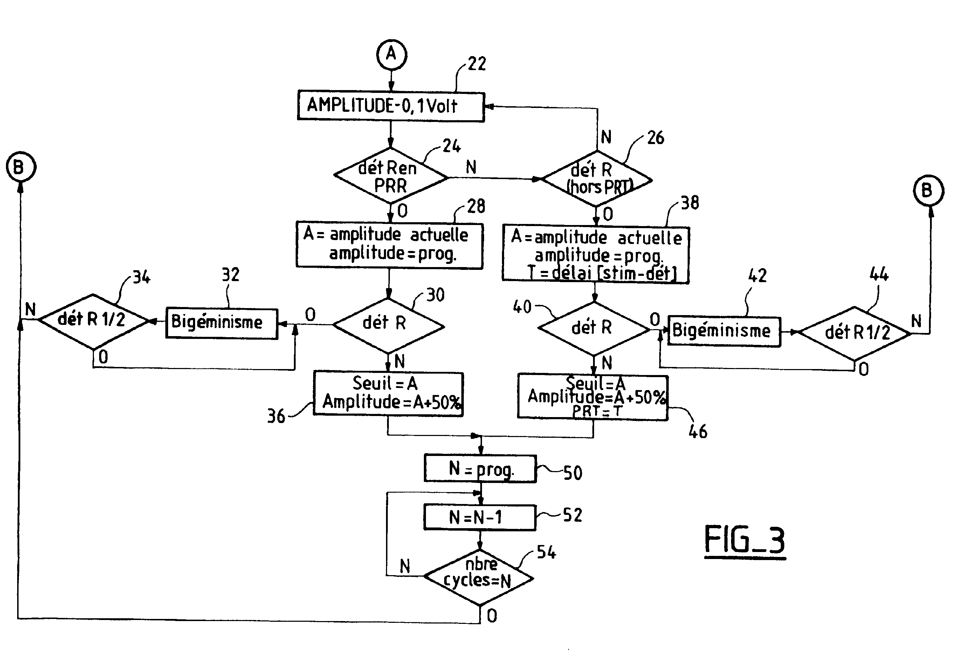

On va maintenant décrire le déroulement du procédé d'ajustement de l'amplitude de stimulation selon l'invention, en référence aux organigrammes des figures 2 et 3.

Dans une phase d'initialisation (étape 10), avec délivrance d'une énergie programmée ou maximale, le stimulateur déplace progressivement vers la gauche (avec les conventions de la figure 1) la frontière F entre période réfractaire absolue et période réfractaire relative (étapes 12 et 14), à partir d'une durée initiale de période réfractaire totale couvrant le temps de récupération des amplificateurs et la durée de la dépolarisation, durée typiquement initialisée à une valeur supérieure 150 ms.

Par ailleurs, on fait en sorte que le test ne soit effectué que si la fréquence sinusale est comprise entre la fréquence de base programmée et cette même fréquence plus 10 % (par exemple), c'est-à-dire que l'on se trouve dans une situation de repos du patient, précaution en elle-même classique dans les algorithmes de ce type.

Dès qu'une détection apparaît dans l'intervalle de la période réfractaire relative (étape 14), on mémorise le délai entre stimulation et détection comme étant le temps maximal de dépolarisation synchrone des deux sites, ou bien le temps de récupération de l'amplificateur si ce dernier est plus long (étape 16). La durée de la période réfractaire absolue, c'est-à-dire, en d'autres termes, la position de la frontière F, reste alors figée à une valeur légèrement supérieure au temps mesuré ; la période correspondant à la différence entre la période réfractaire absolue programmée et celle déterminée dans la phase d'initialisation devient alors la période réfractaire relative.

Une seconde phase de recherche de capture (étape 18) commence alors. Cette phase ne débutera cependant que si l'on est certain que le patient a été libre de toute activité extrasystolique pendant les dix derniers cycles (étape 20).

On diminue alors progressivement l'amplitude de stimulation (étape 22) de manière itérative jusqu'à détecter celui des deux sites ventriculaires qui ne produit plus de capture. Pour cette diminution progressive, il est important de pouvoir disposer d'un pas faible, par exemple de 0,1 V.

Ainsi, si une détection ventriculaire survient en période réfractaire relative, après la période réfractaire absolue (étape 24), alors on a détecté le seuil le plus élevé des deux cavités. En d'autres termes, tant que la capture des deux ventricules est assurée, on ne détecte rien dans la période réfractaire relative. En revanche, si une dépolarisation est détectée dans la période réfractaire relative, c'est alors le signe qu'il y a eu perte de capture dans l'un des deux ventricules et cette détection est le signe d'une dépolarisation conduite d'un ventricule à l'autre.

Dans le cycle suivant le stimulateur rétablit l'amplitude programmée ou maximale (étape 28):
a) si une détection R asynchrone survient (étape 30)- c'est-à-dire que même quand on change l'amplitude de l'impulsion il se produit une seconde dépolarisation indépendante de cette amplitude -, le patient est en bigéminisme (étape 32), c'est-à-dire qu'il y a alternance d'un cycle d'activité normale et d'un cycle d'activité anormale ; il est dans ce cas fort probable que la détection du cycle précédent n'était pas liée au seuil de capture, et l'on attend alors la fin de la séquence stimulation-détection (étape 34) avant de reprendre le test de capture (retour à l'étape 18).
b) si aucune détection ne survient dans le ventricule (étape 30), alors le stimulateur a bien trouvé le seuil de capture le plus élevé des deux cavités, et le stimulateur programme l'amplitude de stimulation au seuil trouvé plus une marge de sécurité, 50 % par exemple (étape 36).
c) si la détection est survenue hors de la période réfractaire relative (étapes 24 et 26), et sous réserve de l'absence de bigéminisme (étapes 38 à 44 homologues des étapes 28 à 34), le stimulateur programme l'amplitude de stimulation au seuil détecté plus une marge de sécurité, 50 % par exemple (étape 46). Il peut éventuellement reprogrammer la période réfractaire relative à une durée égale au délai stimulation-détection plus 31 ms, la période réfractaire relative étant ainsi allongée pour éviter tout risque de fausse détection (le délai stimulation-détection T aura été mémorisé à l'étape 38).

Le test de capture reprend ensuite, de la même façon que ci-dessus, après N cycles de stimulation ventriculaire (étapes 50, 52, 54), N étant une valeur préprogrammée, choisie pour correspondre à une durée d'environ six heures.

## Revendications

1. Un dispositif médical implantable actif, notamment un stimulateur cardiaque, défibrillateur et/ou cardioverteur,
ce dispositif étant du type multisite comprenant des électrodes aptes à être placées en un site ventriculaire droit et en un site ventriculaire gauche, et/ou en un site auriculaire droit et en un site auriculaire gauche,
ces électrodes étant reliées à
- un circuit de recueil de signaux cardiaques pour détecter un potentiel de dépolarisation sur le site correspondant ainsi qu'à
- un circuit de double stimulation ventriculaire, et/ou de double stimulation atriale, apte à appliquer si nécessaire des impulsions de stimulation aux ventricules droit et gauche, et/ou respectivement aux oreillettes droite et gauche,
ce dispositif comportant :
- des moyens d'ajustement de l'amplitude de stimulation des deux sites droit et gauche, par rapport à un seuil d'entraînement définissant le niveau minimal d'amplitude permettant la capture d'une dépolarisation consécutive à une impulsion de stimulation antérieure, ces moyens d'ajustement d'amplitude comprenant des moyens de recherche de capture, aptes à diminuer l'amplitude de stimulation jusqu'à détection d'une perte de capture,
dispositif **caractérisé en ce qu'**il comprend en outre :
- des moyens pour fixer une période réfractaire totale (PRT) comportant une période réfractaire absolue (PRA), pendant laquelle est inhibée toute détection d'une dépolarisation, suivie d'une période réfractaire relative (PRR),
- et des moyens aptes à détecter une perte de capture sur l'un des deux sites, en réponse à la détection d'une dépolarisation pendant la période réfractaire relative.

2. Le dispositif de la revendication 1, comprenant des moyens pour ajuster l'instant (F) de début de la période réfractaire relative.

3. Le dispositif de la revendication 2, dans lequel l'ajustement de l'instant de début de la période réfractaire relative est opéré par accroissement progressif de la durée de cette période réfractaire relative sur plusieurs cycles successifs jusqu'à détection d'une dépolarisation dans cette période réfractaire relative.

4. Le dispositif de la revendication 3, dans lequel la durée de de la période réfractaire totale est constante.

5. Le dispositif de la revendication 4, dans lequel la durée initiale de la période réfractaire relative est nulle.

6. Le dispositif de la revendication 1, dans lequel il est prévu des moyens de détection préalable d'absence d'activité extrasystolique, conditionnant la mise en oeuvre des moyens d'ajustement de l'amplitude de stimulation.

7. Le dispositif de la revendication 6, dans lequel les moyens de détection préalable d'absence d'activité extrasystolique comprennent des moyens pour évaluer le temps maximal de dépolarisation synchrone des deux sites ventriculaires et/ou des deux sites atriaux.

8. Le dispositif de la revendication 1, comprenant en outre des moyens de détection de bigéminisme, propres à inhiber l'ajustement de l'amplitude de stimulation même en cas de détection d'une dépolarisation pendant la période réfractaire relative.

## Claims

1. An active implantable medical device, in particular a pacemaker, defibrillator and/or cardioverter,
this device being of the multisite type comprising electrodes that can be placed at a right ventricular site and a left ventricular site, and/or a right atrial site and a left atrial site,
these electrodes being connected to:
- a cardiac signal collection circuit to detect a potential of depolarisation on the corresponding site, and to
- a double ventricular and/or double atrial stimulation circuit capable of applying, if necessary, stimulation pulses to the right and left ventricle, and/or respectively to the right and left atrium,
this device comprising:
- means for adjusting the amplitude of stimulation of the two sites, right and left, in relation to a training threshold defining the minimal amplitude level allowing the capture of a depolarisation consecutive to an earlier stimulation pulse,
this amplitude adjustment means comprising a means for searching a capture, which is capable of reducing the amplitude of stimulation up to the detection of a loss of capture,
a device **characterised in that** it further comprises:
- means for fixing a total refractory period (PRT) comprising an absolute refractory period (PRA) during which all detection of a depolarisation is inhibited, followed by a relative refractory period (PRR),
- and means capable of detecting a loss of capture at one of the two sites, in response to the detection of a depolarisation during the relative refractory period.

2. The device of claim 1, comprising means to adjust the instant (F) of the beginning of the relative refractory period.

3. The device of claim 2, wherein the adjustment of the instant of the beginning of the relative refractory period is done by a progressive increase of the duration of this relative refractory period over several successive cycles until there is a detection of a depolarisation within said relative refractory period.

4. The device of claim 3, wherein the duration of the total refractory period is constant.

5. The device of claim 4, wherein the initial duration of the relative refractory period is zero.

6. The device of claim 1, comprising means for preliminary detection of the absence of extrasystolic activity, conditioning the implementation of the means for adjusting the stimulation amplitude.

7. The device of claim 6, wherein the means for preliminary detection of the absence of extrasystolic activity comprises means for evaluating the maximum time of synchronous depolarisation of the two ventricular sites and/or the two atrial sites.

8. The device of claim 1, further comprising means for detecting bigeminy, capable of inhibiting the adjustment of the stimulation amplitude even in the case of detection of a depolarisation during the relative refractory period.

## Patentansprüche

1. Aktive implantierbare medizinische Vorrichtung, insbesondere Herzschrittmacher, Defibrillator und/oder Kardioverter,
wobei diese Vorrichtung vom Multisite-Typ ist, mit Elektroden, die geeignet sind, an einem rechten Herzkammersitz und einem linken Herzkammersitz, und/oder an einem rechten Herzvorhofsitz und einem linken Herzvorhofsitz angeordnet zu werden,
wobei diese Elektroden verbunden sind mit:
- einer Schaltung zur Aufnahme von Herzsignalen zur Erfassung eines Depolarisationspotentials am entsprechenden Sitz, sowie mit
- einer Schaltung zur doppelten Herzkammerstimulation, und/oder zur doppelten Herzvorhofstimulation, die geeignet ist, falls erforderlich, an die rechte und linke Herzkammer, und/oder jeweils an den rechten und linken Herzvorhof Stimulationsimpulse abzugeben,
wobei diese Vorrichtung folgendes umfasst:
- Mittel zur Anpassung der Amplitude der Stimulation der zwei Sitze rechts und links, im Verhältnis zu einer Trainingsschwelle, die das Niveau geringster Amplitude definiert, welches die Erfassung einer sich an einen vorherigen Stimulationsimpuls anschließenden Depolarisation erlaubt,
wobei diese Mittel zur Anpassung der Amplitude Mittel zur Erzielung einer Erfassung umfassen, die geeignet sind, die Stimulationsamplitude bis zur Detektion eines Ausfalls der Erfassung zu verringern,
Vorrichtung, die **dadurch gekennzeichnet ist, dass** sie weiterhin folgendes umfasst:
- Mittel um einen refraktären Gesamtzeitraum (PRT) festzulegen, der einen refraktären Absolutzeitraum (PRA), während welchem jegliche Erfassung einer Depolarisation unterdrückt ist, umfasst, gefolgt von einem refraktären Relativzeitraum (PRR),
- und Mittel, die geeignet sind, den Ausfall einer Erfassung an einem der zwei Sitze zu detektieren, in Antwort auf die Erfassung einer Depolarisation während des refraktären Relativzeitraums.

2. Vorrichtung nach Anspruch 1, mit Mitteln zur Anpassung des Zeitpunkts (F) des Beginns des refraktären Relativzeitraums.

3. Vorrichtung nach Anspruch 2, bei welcher die Anpassung des Zeitpunkts des Beginns des refraktären Relativzeitraums durch die progressive Erhöhung der Dauer dieses refraktären Relativzeitraums über mehrere aufeinander folgende Zyklen durchgeführt wird, bis zur Erfassung einer Depolarisation während dieses refraktären Relativzeitraums.

4. Vorrichtung nach Anspruch 3, bei welcher die Dauer des refraktären Gesamtzeitraums konstant ist.

5. Vorrichtung nach Anspruch 4, bei welcher die ursprüngliche Dauer des refraktären Relativzeitraums gleich null ist.

6. Vorrichtung nach Anspruch 1, bei welcher Mittel zur vorhergehenden Erfassung einer Abwesenheit einer Extrasystolen-Aktivität vorgesehen sind, welche die Umsetzung der Mittel zur Anpassung der Amplitude der Stimulation bedingen.

7. Vorrichtung nach Anspruch 6, bei welcher die Mittel zur vorhergehenden Erfassung einer Abwesenheit einer Extrasystolen-Aktivität Mittel zur Abschätzung der Maximalzeit der synchronen Depolarisation der zwei Herzkammersitze und/oder der zwei Herzvorhofsitze umfassen.

8. Vorrichtung nach Anspruch 1, weiterhin mit Mitteln zur Erfassung einer Bigeminie, die in der Lage sind, die Anpassung der Amplitude der Stimulation selbst im Falle der Erfassung einer Depolarisation während des refraktären Relativzeitraums zu unterdrücken.
